(19) Europäisches Patentamt
European Patent Office
Office européen des brevets

(11) Publication number: **0 253 503 B1**

(12) # EUROPEAN PATENT SPECIFICATION

(45) Date of publication of patent specification: **11.12.91**

(51) Int. Cl.5: **C07C 233/01**, C07C 255/58, C07C 317/14, C07C 317/02, C07C 323/00, A61K 31/165, A61K 31/275

(21) Application number: **87305271.6**

(22) Date of filing: **12.06.87**

(54) **Substituted anilides having antiandrogenic properties.**

(30) Priority: **18.07.86 GB 8617653**

(43) Date of publication of application: **20.01.88 Bulletin 88/03**

(45) Publication of the grant of the patent: **11.12.91 Bulletin 91/50**

(84) Designated Contracting States: **CH DE FR GB IT LI**

(56) References cited:
**EP-A- 0 040 932**
**EP-A- 0 100 172**
**EP-A- 0 184 822**
**US-A- 4 536 346**

**CHEMICAL ABSTRACTS, vol. 92, no. 21, 26th May 1980, page 587, no. 180461g, Columbus, Ohio, US; R. CHIRON et al.: "An investigation of the interaction between the different factors influencing the intramolecular hydrogen bonding of gamma- and delta-keto amides"**

(73) Proprietor: **IMPERIAL CHEMICAL INDUSTRIES PLC**
**Imperial Chemical House, Millbank**
**London SW1P 3JF(GB)**

(72) Inventor: **Tucker, Howard**
**32 Millers Meadow Rainow**
**Macclesfield Cheshire(GB)**

(74) Representative: **Slatcher, Reginald Peter et al**
**Imperial Chemical Industries PLC Legal Department: Patents PO Box 6**
**Welwyn Garden City Herts, AL7 1HD(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to new amide derivatives and more particularly it relates to novel acylanilides which possess antiandrogenic properties.

It is known from European Patent Applications Nos. 0040932 and 0100172 that certain acylanilide derivatives possess antiandrogenic properties.

It is also known from European Patent Application No. 0184822 that certain acylanilide derivatives possess activity as cerebral metabolism stimulants or nootropic agents.

According to the invention there is provided an acylanilide of the formula:

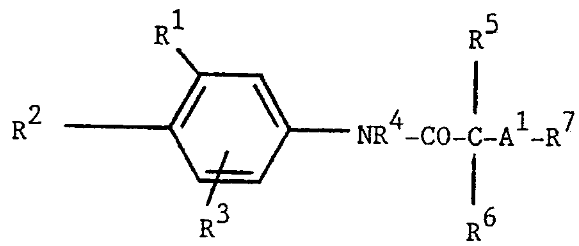

wherein $R^1$ is cyano, carbamoyl, nitro, fluoro, chloro, bromo, iodo or hydrogen, or alkyl, alkoxy, alkanoyl, alkylthio, alkylsulphinyl, alkylsulphonyl, perfluoroalkyl, perfluoroalkylthio, perfluoroalkylsulphinyl or perfluoroalkylsulphonyl each of up to 4 carbon atoms, or phenylthio, phenylsulphinyl or phenylsulphonyl;
wherein $R^2$ is cyano, carbamoyl, nitro, fluoro, chloro, bromo or iodo, or alkanoyl, alkylthio, alkylsulphinyl, alkylsulphonyl, perfluoroalkyl, perfluoroalkylthio, perfluoroalkylsulphinyl or perfluoroalkylsulphonyl each of up to 4 carbon atoms, or phenylthio, phenylsulphinyl or phenylsulphonyl;
wherein $R^3$ is hydrogen or halogen;
wherein $R^4$ is hydrogen or alkyl of up to 4 carbon atoms, or is joined to $R^5$ as stated below;
wherein $R^5$ is hydroxy or alkoxy or acyloxy each of up to 15 carbon atoms, or is joined to $R^4$ to form an oxycarbonyl group such that together with the -N-CO-C- part of the molecule it forms an oxazolidinedione group;
wherein $R^6$ is alkyl or halogenoalkyl each of up to 4 carbon atoms;
wherein $A^1$ is straight-chain alkylene of up to 10 carbon atoms, or alkenylene or alkynylene each of 2 to 10 carbon atoms;
and wherein $R^7$ is selected from phenyl which bears one, two or three substituents selected from hydrogen, halogen, nitro, hydroxy, carboxy, carbamoyl and cyano, alkyl, alkoxy, alkanoyl, alkylthio, alkylsulphinyl, alkylsulphonyl, perfluoroalkyl, perfluoroalkylthio, perfluoroalkylsulphinyl, perfluoroalkylsulphonyl, alkoxycarbonyl and N-alkylcarbamoyl each of up to 4 carbon atoms, phenyl, phenylthio, phenylsulphinyl and phenylsulphonyl, naphthyl and 5- or 6- membered saturated or unsaturated heterocyclic which contains one, two or three heteroatoms selected from oxygen, nitrogen and sulphur, which heterocyclic may be a single ring or may be fused to a benzo-ring, and which heterocyclic is unsubstituted or bears one or two halogen, cyano or amino, alkyl, alkoxy, alkylthio, alkylsulphinyl or alkylsulphonyl each of up to 4 carbon atoms, oxy or hydroxy substituents, or which if sufficiently saturated may bear one or two oxo substituents.

It will be observed that the acylanilide derivative of the invention possesses an asymmetric carbon atom, namely the carbon atom which bears the substituents $R^5$ and $R^6$, and it can therefore exist in racemic and optically-active forms. It is to be understood that this invention encompasses the racemic form of the acylanilide derivative and any optically-active form which possesses antiandrogenic activity, it being a matter of common general knowledge how a racemic compound may be resolved into its optically-active forms and how any antiandrogenic activity present in any of these forms may be determined.

A suitable value for $R^1$ or $R^4$ when it is alkyl, or for an alkyl substituent in $R^7$ when $R^7$ is phenyl or heterocyclic substituted by alkyl is, for example, methyl or ethyl.

A suitable value for $R^1$ when it is alkoxy or for an alkoxy substituent in $R^7$ when $R^7$ is phenyl or heterocyclic substituted by alkoxy is, for example, methoxy or ethoxy.

A suitable value for $R^1$ or $R^2$ when it is alkanoyl, or for an alkanoyl substituent in $R^7$ when $R^7$ is phenyl substituted by alkanoyl is, for example, formyl or acetyl.

A suitable value for $R^1$ or $R^2$ when it is alkylthio, alkylsulphinyl, alkylsulphonyl, perfluoroalkyl, perfluoroalkylthio, perfluoroalkylsulphinyl or perfluoroalkylsulphonyl, or for such a substituent in $R^7$ when $R^7$ is phenyl or heterocyclic bearing such a substituent is, for example, methylthio, ethylthio, methylsulphinyl,

methylsulphonyl, trifluoromethyl, pentafluoroethyl, trifluoromethylthio, trifluoromethylsulphinyl or trifluoromethylsulphonyl.

A suitable value for $R^3$ when it is halogen, or for a halogen substituent in $R^7$ when $R^7$ is phenyl or heterocyclic substituted by halogen, is fluoro, chloro, bromo or iodo.

$R^3$ is preferably hydrogen or chloro, especially hydrogen.

$R^4$ is preferably hydrogen.

A suitable value for an alkoxycarbonyl or N-alkylcarbamoyl substituent in $R^7$ when $R^7$ is phenyl bearing such a substituent is, for example, methoxycarbonyl, ethoxycarbonyl or N-methylcarbamoyl.

A suitable value for $R^5$ when it is alkoxy is, for example, methoxy, ethoxy, propyloxy, n-butyloxy or decyloxy.

A suitable value for $R^5$ when it is acyloxy is, for example, alkanoyl or aroyl each of up to 15 carbon atoms, for example acetoxy, propionyloxy, decanoyloxy, dodecanoyloxy or benzoyloxy.

$R^5$ is preferably hydroxy.

A suitable value for $R^6$ when it is alkyl or halogenoalkyl is, for example, methyl, ethyl, n-propyl, fluoromethyl, difluoromethyl, trifluoromethyl, pentafluoroethyl, chloromethyl, dichloromethyl or trichloromethyl. $R^6$ is preferably trifluoromethyl.

A suitable value for $A^1$ when it is alkylene is, for example, methylene, ethylene, trimethylene or tetramethylene.

A suitable value for $A^1$ when it is alkenylene or alkynylene is, for example, vinylene (-CH=CH-), prop-1-enylene (-CH=CH-$CH_2$-), 2-methylprol-1-enylene

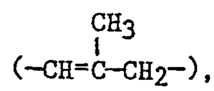

ethynylene (-C≡C-), prop-1-ynylene (-C≡C-$CH_2$-) or prop-2-ynylene (-$CH_2$C≡C-).

A suitable value for $R^7$ when it is heterocyclic is, for example, furyl, thienyl, pyrrolyl, pyridyl, imidazolyl, thiazolyl, thiadiazolyl, benzimidazolyl, indolyl, benzothienyl, benzofuryl, quinolyl, isoquinolyl or 1,2-dihydro-2-oxoquinolyl.

A preferred combination of values for $R^1$ and $R^2$ is as follows:-

| $R^1$ | $R^2$ |
|---|---|
| trifluoromethyl | nitro |
| trifluoromethyl | cyano |
| chloro | chloro |
| chloro | nitro |
| chloro | cyano |
| cyano | cyano |
| nitro | cyano |
| ethoxy | nitro |
| chloro | methylsulphonyl |

A preferred acylanilide of the invention has the formula stated above wherein $R^1$ and $R^2$, which may be the same or different, each is cyano, nitro, trifluoromethyl, methylthio, methylsulphinyl, methylsulphonyl or chloro, $R^3$ and $R^4$ are both hydrogen, $R^5$ is hydroxy, $R^6$ is methyl or trifluoromethyl, $A^1$ is methylene, ethylene, trimethylene or tetramethylene and $R^7$ is phenyl which is unsubstituted or which bears one fluoro, chloro, hydroxy, methyl, methylthio, methylsulphinyl or methylsulphonyl substitutent.

A further preferred acylanilide of the invention, which possesses, in addition to antiandrogenic properties, progestational, antiprogestational or both progestational and antiprogestational properties, has the formula stated above wherein $R^1$ is cyano, fluoro, hydrogen, ethoxy, methylsulphonyl or trifluoromethyl, wherein $R^2$ is cyano or nitro, wherein $R^3$ and $R^4$ are both hydrogen and $R^5$ is hydroxy, wherein $R^6$ is trifluoromethyl, wherein $A^1$ is methylene, ethylene, trimethylene or tetramethylene and wherein $R^7$ is phenyl which is unsubstituted or bears one substituent selected from fluoro, chloro and methyl.

An especially preferred acylanilide of the invention, which possesses, in addition to antiandrogenic properties, progestational or both progestational and antiprogestational properties, has the formula:-

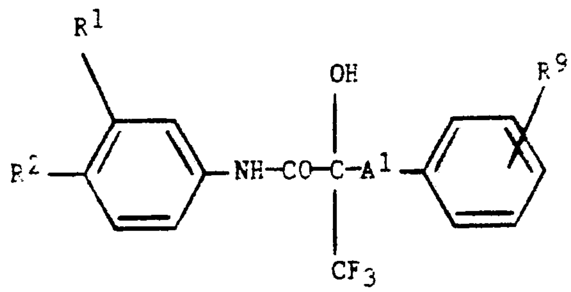

wherein the specific values of $R^1$, $R^2$, $A^1$ and $R^9$ are shown in the following table:-

| $R^1$ | $R^2$ | $A^1$ | $R^9$ |
|---|---|---|---|
| $CF_3$ | CN | $-CH_2-$ | H |
| $CF_3$ | $NO_2$ | $-CH_2-$ | H |
| $CF_3$ | $NO_2$ | $-CH_2-$ | 4-$CH_3$ |
| $CF_3$ | $NO_2$ | $-CH_2-$ | 2-Cl |
| $CF_3$ | $NO_2$ | $-CH_2-$ | 3-Cl |
| $CF_3$ | $NO_2$ | $-CH_2-$ | 4-Cl |
| $CF_3$ | $NO_2$ | $-CH_2-$ | 2-F |
| $CF_3$ | $NO_2$ | $-CH_2-$ | 3-F |
| Cl | $NO_2$ | $-CH_2-$ | H |
| Cl | CN | $-CH_2-$ | 2-Cl |
| Cl | CN | $-CH_2-$ | 3-Cl |
| Cl | CN | $-CH_2-$ | 4-Cl |
| Cl | CN | $-CH_2-$ | 3-F |
| $C_2H_5O$ | $NO_2$ | $-CH_2-$ | H |
| $CH_3SO_2$ | $NO_2$ | $-CH_2-$ | H |
| $CF_3$ | $NO_2$ | $-CH_2CH_2-$ | H |
| $CF_3$ | $NO_2$ | $-(CH_2)_4-$ | H |
| $CF_3$ | $NO_2$ | $-CH_2-$ | 4-F |

A further especially preferred acylanilide of the invention, which possesses, in addition to antiandrogenic properties, antiprogestational or both antiprogestational and progestational properties, has the formula:-

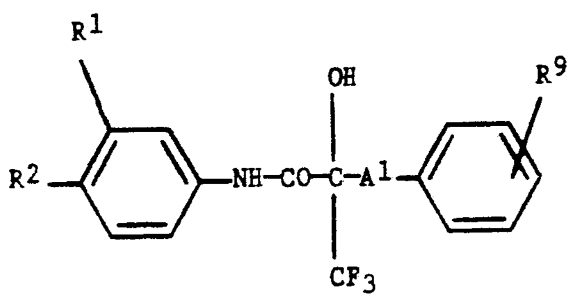

wherein the specific values of $R^1$, $R^2$, $A^1$ and $R^9$ are shown in the following table:-

| $R^1$ | $R^2$ | $A^1$ | $R^9$ |
|---|---|---|---|
| Cl | CN | $-CH_2-$ | H |
| $CF_3$ | CN | $-CH_2-$ | H |
| $CF_3$ | CN | $-CH_2-$ | 2-Cl |
| $CF_3$ | CN | $-CH_2-$ | 2-F |
| $CF_3$ | CN | $-CH_2-$ | 3-F |
| $CF_3$ | CN | $-CH_2-$ | 4-F |
| $CF_3$ | $NO_2$ | $-CH_2-$ | H |
| $CF_3$ | $NO_2$ | $-CH_2-$ | 4-Cl |
| $CF_3$ | $NO_2$ | $-CH_2-$ | 2-F |
| $CF_3$ | $NO_2$ | $-CH_2-$ | 3-F |
| Cl | $NO_2$ | $-CH_2-$ | H |
| Cl | CN | $-CH_2-$ | 2-Cl |
| Cl | CN | $-CH_2-$ | 4-Cl |
| Cl | CN | $-CH_2-$ | 2-F |
| Cl | CN | $-CH_2-$ | 3-F |
| Cl | CN | $-CH_2-$ | 4-F |
| F | CN | $-CH_2-$ | H |
| F | CN | $-CH_2-$ | 4-F |
| CN | CN | $-CH_2-$ | H |
| H | CN | $-CH_2-$ | H |
| H | $NO_2$ | $-CH_2-$ | H |
| H | $NO_2$ | $-CH_2-$ | 2-Cl |
| $CF_3$ | $NO_2$ | $-(CH_2)_3-$ | H |
| $CF_3$ | $NO_2$ | $-CH_2-$ | 4-F |

Specific acylanilides of the invention are hereinafter described in the Examples.

Particularly active compounds are 3-chloro-4-cyano-N-(2-hydroxy-3-p-methanesulphonylphenyl-2-trifluoromethylpropionyl)aniline;
3-chloro-4-cyano-N-(3-p-fluorophenyl-2-hydroxy-2-trifluoromethylpropionyl)aniline;
4-cyano-3-trifluoromethyl-N-(3-p-chlorophenyl-2-hydroxy-2-trifluoromethylpropionyl)aniline;
4-cyano-3-trifluoromelthyl-N-(2-hydroxy-3-phenyl-2-trifluoromethylpropionyl)aniline;
4-nitro-3-triluoromethyl-N-(2-hydroxy-3-phenyl-2-trifluoromethylpropionyl)aniline;
4-nitro-3-trifluoromethyl-N-(3-p-chlorophenyl-2-hydroxy-2-trifluoromethylpropionyl)aniline;
4-nitro-3-trifluoromethyl-N-(3-p-fluorophenyl-2-hydroxy-2-trifluoromethylpropionyl)aniline;
4-nitro-N-(3-p-fluorophenyl-2-hydroxy-2-trifluoromethylpropionyl)aniline;
4-cyano-N-(2-hydroxy-2-trifluoromethyl-4-phenylbutyryl)aniline;
4-nitro-3-trifluoromethyl-N-(3-o-chlorophenyl-2-hydroxy-2-trifluoromethylpropionyl)aniline;
4-nitro-3-trifluoromethyl-N-(3-m-fluorophenyl-2-hydroxy-2-trifluoromethylpropionyl)anilineor
4-cyano-3-trifluoromethyl-N-(3-p-fluorophenyl-2-hydroxy-2-trifluoromethylpropionyl)aniline.

The acylanilides of the invention may be manufactured by any chemical process known to be suitable for the manufacture of chemically-analogous compounds.

A preferred process for the manufacture of an acylanilide of the invention comprises the reaction of an amine of the formula:-

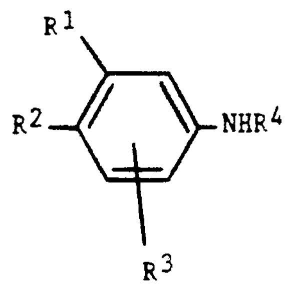

wherein $R^1$, $R^2$, $R^3$ and $R^4$ have the meanings stated above, with an acid of the formula:-

$HO_2C-CR^5R^6-A^1-R^7$

wherein $R^5$, $R^6$, $R^7$ and $A^1$ have the meanings stated above, or with a reactive derivative of said acid.

A suitable reactive derivative of an acid is, for example, an acid anhydride, or an acyl halide, for example an acyl chloride, or a lower alkyl ester of said acid, for example the methyl or ethyl ester.

Preferably the reaction is carried out in N,N-dimethylacetamide solution using an acyl chloride (prepared from the acid and thionyl chloride) as reactant.

An acylanilide of the invention wherein $R^4$ and $R^5$ are joined together to form a carbonyl-oxy group, that is, an oxazolidinedione, may be prepared by the reaction of an isocyanate of the formula:-

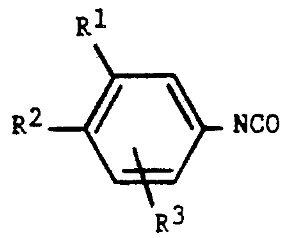

wherein $R^1$, $R^2$ and $R^3$ have the meanings stated above, with an ester of the formula:-

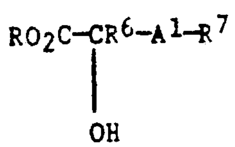

wherein $R^6$, $R^7$ and $A^1$ have the meanings stated above, and wherein R is alkyl of up to 6 carbon atoms, for example methyl or ethyl. This reaction is preferably carried out in an organic solvent, for example diethyl ether, at laboratory temperature.

An acylanilide of the invention wherein $R^7$ is heterocyclic and $A^1$ is methylene may be prepared by the reaction of an epoxide of the formula:-

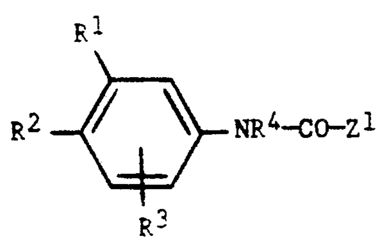

wherein $R^1$, $R^2$, $R^3$ and $R^4$ have the meanings stated above and wherein $Z^1$ has the formula:-

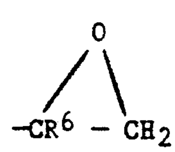

wherein $R^6$ has the meaning stated above, with a heterocycle or a reactive derivative thereof of the formula $R^7$-M wherein $R^7$ has the meaning stated immediately above and M is a metal radical. The reaction is preferably carried out in an inert diluent or solvent, for example diethyl ether or tetrahydrofuran at or near laboratory temperature, for example at between 0 °C and 50 °C.

The epoxide used as starting material may be obtained by the epoxidation, for example with a peracid,

of the corresponding unsaturated acylanilide.

A suitable reactive derivative of a heterocycle of the formula $R^7$-M is, for example, an alkali metal salt of the heterocycle, for example the lithium or sodium salt, which may be prepared by the reaction of the heterocycle with, for example, an alkali metal alkyl, for example butyllithium.

The last-mentioned reaction is preferably carried out in an inert solvent, for example diethyl ether or tetrahydrofuran, at a low temperature, for example at between -30° and -80° C.

An acylanilide of the invention wherein $R^5$ is hydroxy may be prepared by the hydrolysis of the corresponding acylanilide wherein $R^5$ is acyloxy, and an acylanilide of the invention wherein $R^5$ is hydroxy and $R^4$ is hydrogen may be prepared by the hydrolysis of the corresponding oxazolidinedione, which may be prepared as described above.

An acylanilide of the invention wherein $R^4$ is alkyl may be prepared by the alkylation of the corresponding acylanilide wherein $R^4$ is hydrogen.

An acylanilide of the invention wherein $R^5$ is acyloxy may be prepared by the acylation of the corresponding acylanilide wherein $R^5$ is hydroxy.

An oxazolidinedione of the invention, wherein $R^4$ and $R^5$ are joined together to form a carbonyl-oxy group, may be prepared by the reaction of the corresponding acylanilide wherein $R^4$ is hydrogen and $R^5$ is hydroxy with phosgene ($COCl_2$).

An acylanilide of the invention wherein one or more of $R^1$, $R^2$ and a substituent in the phenyl or heterocyclic group $R^7$ is alkylsulphinyl, perfluoroalkylsulphinyl or phenylsulphinyl, or is alkylsulphonyl, perfluoroalkylsulphonyl or phenylsulphonyl, may be prepared by the oxidation of the corresponding acylanilide wherein one or more of $R^1$, $R^2$ and a substituent in the phenyl or heterocyclic group $R^7$ is alkylthio, perfluoroalkylthio or phenylthio, respectively. The oxidising agent and conditions used will determine whether a sulphinyl or sulphonyl compound is obtained. Thus oxidation with sodium metaperiodate in methanol solution at or below laboratory temperature will generally convert a thio compound into the corresponding sulphinyl compound; and oxidation with a peracid, for example m-chloroperbenzoic acid in methylene chloride solution at or above laboratory temperature will generally convert a thio compound into the corresponding sulphonyl compound.

A racemic acylanilide of the invention wherein $R^5$ is hydroxy may be separated into its optical isomers by forming an ester of the hydroxy group $R^5$ with an optically-active acid, for example (-)-camphanic acid, separating the diastereoisomeric esters thus obtained, by fractional crystallisation or, preferably, by flash-chromatography, and then hydrolysis of each separate ester to the alcohol.

As stated above, an acylanilide of the invention possesses antiandrogenic properties as demonstrated by its ability to decrease the weight of the seminal vesicles of a mature male rat when administered orally for 4 successive days. An acylanilide of the invention may be used in the treatment of, for example, malignant or benign prostatic disease or of androgen dependent disease conditions, such as acne, hirsutism or seborrhoea, in warm-blooded vertebrates including man. It may also be used to improve ovulation in a domestic animal.

A preferred acylanilide of the invention is up to 10 times more active as an antiandrogen than the known, chemically-related antiandrogens flutamide and hydroxyflutamide. At a dose of an acylanilide of the invention which produces antiandrogenic activity in rats no symptoms of toxicity are apparent.

Some of the acylanilides of the invention also possess other hormonal or antihormonal activity, for example progestational or antiprogestational activity, or both such activities.

Any progestational activity possessed by an acylanilide of the invention can be demonstrated by its ability to promote glandular development in the endometrium of an oestrogen-primed immature rabbit, the standard Clauberg assay procedure. An acylanilide of the invention which possesses progestational activity may be used as a contraceptive, and in the treatment of menstrual disorders, such as dysmemorrhea, dysfunctional bleeding and premenstrual tension, and in the treatment of hormone dependent tumours, especially those of the breast or endometrium. It may also be used for the synchronisation of oestrus and for the maintenance of early pregnancy in domestic animals such as cattle. At a dose of an acylanilide of the invention which produces progestational activity in rabbits no symptoms of toxicity are apparent.

Any antiprogestational activity possessed by an acylanilide of the invention can be demonstrated by its ability to terminate by day 16 the pregnancy of a mature female rat when administered subcutaneously twice on day 9 and once on day 10 of the pregnancy. An acylanilide of the invention which possesses antiprogestational activity may be used as a contraceptive, and in the treatment of menstrual disorders, such as dysmenorrhea, dysfunctional bleeding and premenstrual tension, and in the treatment of hormone dependent tumours, especially those of the breast or endometrium. At a dose of an acylanilide of the invention which produces antiprogestational activity in rats no symptoms of toxicity are apparent.

The acylanilide of the invention may be administered to a warm blooded animal in the form of a

pharmaceutical or veterinary composition which comprises the acylanilide in association with a pharmaceutically acceptable diluent or carrier.

The composition may be in a form suitable for oral dosage, as a tablet, capsule, aqueous or oily solution or suspension or emulsion. It may alternatively be in the form of a sterile solution or suspension suitable for parenteral administration, or be in the form of an ointment or lotion for topical administration or be in the form of a suppository for anal or vaginal administration.

The composition may additionally contain one or more drugs selected from anti-oestrogens, for example tamoxifen; progestins, for example medroxyprogesterone acetate; inhibitors of gonadotrophin secretion, for example danazol; cytotoxic agents, for example cyclophosphamide; antibiotics, for example penicillin or oxytetracyclin; and anti-inflammatory agents, for example, especially for topical use, fluocinolone acetonide.

The acylanilide of the invention will normally be administered to a warm-blooded animal at a dose of between 0.1 mg. And 125 mg. Per kg. bodyweight.

The invention is illustrated but not limited by the following Examples:-

## Example 1

Thionyl chloride (0.73 ml.) Was added to a stirred solution of 2-hydroxy-3-phenyl-2-trifluoromelthylpropionic acid (2.34 g.) In N,N-dimethylacetamide (40 ml.) which was cooled to -15°C., at such a rate that that temperature was maintained, and the mixture was stirred at that temperature for 15 minutes. 3-Chloro-4-cyanoaniline (1.5 g.) Was added, the mixture was stirred at -15°C. for 15 minutes and then at laboratory temperature for 15 hours, and was then poured into water (800 ml.). The mixture was extracted six times with diethyl ether (80 ml. each time) and the combined extracts were washed successively (50 ml. portions each time) twice with aqueous 2N-hydrochloric acid, once with saturated aqueous sodium chloride solution, twice with saturated aqueous sodium bicarbonate solution, and again once with saturated aqueous sodium chloride solution, dried over magnesium sulphate and evaporated to dryness under reduced pressure. The residue was purified by chromatography on a silica gel column (Merck 7734) using a 1:1 v/v mixture of ethyl acetate and petroleum ether (b.p. 60-80°C.) as eluant. The product was crystallised from toluene and there was thus obtained 3-chloro-4-cyano-N-(2-hydroxy-3-phenyl-2-trifluoromethylpropionyl)aniline,m.p. 153-154°C.

The 2-hydroxy-3-phenyl-2-trifluoromethylpropionic acid used as starting material was prepared as follows:-

1,1,1-Trifluoro-3-phenylpropan-2-one (8.2 g, obtained by the process described in the Journal of Organic Chemistry, 1967, 32, 1316) was added dropwise to a cooled stirred solution of potassium cyanide (3.2 g.) in water (12 ml.) at such a rate that the temperature of the mixture was maintained at between 0° and 5°C. A 4:1 v/v mixture of water and concentrated sulphuric acid (60 ml.) was added at such a rate to maintain the above temperature, and the mixture was then stirred at laboratory temperature for 15 hours and then extracted three times with diethyl ether (20 ml. each time). The combined extracts were washed three times with water (25 ml. each time), dried overmagnesium sulphate and evaporated to dryness under reduced pressure.

A mixture of the cyanhydrin thus obtained (3.0 g.), concentrated aqueous hydrochloric acid (24 ml.) and acetic acid (6 ml.) was heated in a sealed tube at 110°C. for 6 hours, cooled and poured onto ice. The aqueous mixture was extracted four times with diethyl ether (25 ml. each time) and the combined ethereal solutions were extracted twice with saturated aqueous sodium bicarbonate solution (40 ml. each time). The combined extracts were acidified with aqueous hydrochloric acid and then extracted twice with diethyl ether (40 ml. each time). The combined extracts were dried over magnesium sulphate and evaporated to dryness and the residue was crystallised from cyclohexane. There was thus obtained 2-hydroxy-3-phenyl-2-trifluoromethylpropionic acid, m.p. 123-124°C.

## Example 2

The process described in Example I was repeated except that the appropriate aniline and the appropriate 2-hydroxyphenylalkanoic acid were used as starting materials. There were thus obtained the compounds described in the following table:-

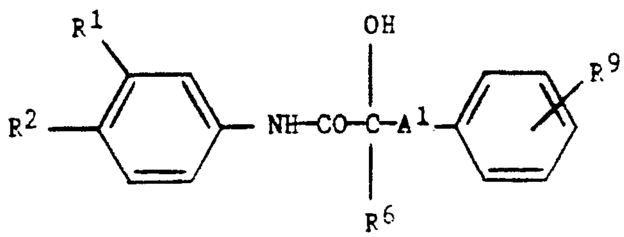

| $R^1$ | $R^2$ | $R^6$ | $A^1$ | $R^9$ | m.p. (°C.) |
|---|---|---|---|---|---|
| $CF_3$ | CN | $CF_3$ | $-CH_2-$ | - | 168-170 |
| $CF_3$ | CN | $CF_3$ | $-CH_2-$ | 2-Cl | 104-105 |
| $CF_3$ | CN | $CF_3$ | $-CH_2-$ | 3-Cl | 144-145 |
| $CF_3$ | CN | $CF_3$ | $-CH_2-$ | 4-Cl | 182-184 |
| $CF_3$ | CN | $CF_3$ | $-CH_2-$ | 2-F | 139-141 |
| $CF_3$ | CN | $CF_3$ | $-CH_2-$ | 3-F | 148-149 |
| $CF_3$ | CN | $CF_3$ | $-CH_2-$ | 4-F | 161-163 |
| $CF_3$ | CN | $CF_3$ | $-CH_2-$ | $4-SCH_3$ | (oil) |
| $CF_3$ | $NO_2$ | $CF_3$ | $-CH_2-$ | - | 119-122 |
| $CF_3$ | $NO_2$ | $CF_3$ | $-CH_2-$ | $4-CH_3$ | 164-166 |
| $CF_3$ | $NO_2$ | $CF_3$ | $-CH_2-$ | 2-Cl | 106-107 |
| $CF_3$ | $NO_2$ | $CF_3$ | $-CH_2-$ | 3-Cl | 161 |
| $CF_3$ | $NO_2$ | $CF_3$ | $-CH_2-$ | 4-Cl | 172-174 |
| $CF_3$ | $NO_2$ | $CF_3$ | $-CH_2-$ | 2,6-diCl | 96-98 |
| $CF_3$ | $NO_2$ | $CF_3$ | $-CH_2-$ | 2-F | 122-123 |
| $CF_3$ | $NO_2$ | $CF_3$ | $-CH_2-$ | 3-F | 175-176 |
| $CF_3$ | $NO_2$ | $CF_3$ | $-CH_2-$ | 4-F | 139-140 |
| $CF_3$ | $NO_2$ | $CF_3$ | $-CH_2-$ | 4-OH | 172-173 |

/Continued...

| $R^1$ | $R^2$ | $R^6$ | $A^1$ | $R^9$ | m.p. (°C.) |
|---|---|---|---|---|---|
| $CF_3$ | $NO_2$ | $CF_3$ | $-CH_2-$ | $4-SCH_3$ | 139–140 |
| Cl | Cl | $CF_3$ | $-CH_2-$ | – | 171–172* |
| Cl | $NO_2$ | $CF_3$ | $-CH_2-$ | – | 160–161 |
| Cl | $NO_2$ | $CF_3$ | $-CH_2-$ | $4-SCH_3$ | (oil) |
| Cl | CN | $CF_3$ | $-CH_2-$ | 2–Cl | 109–111 |
| Cl | CN | $CF_3$ | $-CH_2-$ | 3–Cl | 169–170 |
| Cl | CN | $CF_3$ | $-CH_2-$ | 4–Cl | 174–175* |
| Cl | CN | $CF_3$ | $-CH_2-$ | 3,4–diCl | 180–181 |
| Cl | CN | $CF_3$ | $-CH_2-$ | 2–F | 136–138 |
| Cl | CN | $CF_3$ | $-CH_2-$ | 3–F | 152–153 |
| Cl | CN | $CF_3$ | $-CH_2-$ | 4–F | 153–154 |
| Cl | CN | $CF_3$ | $-CH_2-$ | $4-SCH_3$ | 143–144 |
| F | CN | $CF_3$ | $-CH_2-$ | – | 129–130 |
| F | CN | $CF_3$ | $-CH_2-$ | 4–F | 127–129 |
| CN | CN | $CF_3$ | $-CH_2-$ | – | 153–154 |
| H | CN | $CF_3$ | $-CH_2-$ | – | 163–164* |
| H | CN | $CF_3$ | $-CH_2-$ | 4–Cl | 174–176* |
| $CH_3$ | CN | $CF_3$ | $-CH_2-$ | – | 129–132* |
| H | $NO_2$ | $CF_3$ | $-CH_2-$ | – | 117–118 |
| H | $NO_2$ | $CF_3$ | $-CH_2-$ | 2–Cl | 92–93 |
| H | $NO_2$ | $CF_3$ | $-CH_2-$ | 4–Cl | 143–144 |
| H | $NO_2$ | $CF_3$ | $-CH_2-$ | 4–F | 119–121 |
| $C_2H_5O$ | $NO_2$ | $CF_3$ | $-CH_2-$ | – | 114–115 |
| Cl | $CH_3S$ | $CF_3$ | $-CH_2-$ | – | 185–186 |
| Cl | $CH_3SO_2$ | $CF_3$ | $-CH_2-$ | – | 175–178 |
| H | $CH_3SO_2$ | $CF_3$ | $-CH_2-$ | – | 200–201* |
| $CH_3S$ | CN | $CF_3$ | $-CH_2-$ | – | 169–172 |
| $CH_3SO_2$ | $NO_2$ | $CF_3$ | $-CH_2-$ | – | 192–194 |
| $C_6H_5S$ | $NO_2$ | $CF_3$ | $-CH_2-$ | – | 167–169 |
| $CF_3$ | $NO_2$ | $CH_3$ | $-CH_2-$ | – | 90–92 |
| $CF_3$ | CN | $CH_3$ | $-CH_2-$ | – | 134–135 |
| Cl | $NO_2$ | $CH_3$ | $-CH_2-$ | – | 132–134 |

/Continued...

| $R^1$ | $R^2$ | $R^6$ | $A^1$ | $R^9$ | m.p. (°C.) |
|---|---|---|---|---|---|
| $CF_3$ | $NO_2$ | $CH_2Cl$ | $-CH_2-$ | – | 80–82 |
| $CF_3$ | $NO_2$ | $CH_2C_6H_5$ | $-CH_2-$ | | 130–131 |
| $CF_3$ | $NO_2$ | 2-Thienyl | $-CH_2-$ | – | 118–121* |
| $CF_3$ | $NO_2$ | $CF_3$ | $-CH_2CH_2-$ | – | 131–132 |
| $CF_3$ | CN | $CF_3$ | $-CH_2CH_2-$ | – | 124–125 |
| Cl | Cl | $CF_3$ | $-CH_2CH_2-$ | – | 129–130* |
| Cl | CN | $CF_3$ | $-CH_2CH_2-$ | – | 154 |
| H | CN | $CF_3$ | $-CH_2CH_2-$ | – | 149–152* |
| H | $NO_2$ | $CF_3$ | $-CH_2CH_2-$ | – | 153–155* |
| $CF_3$ | $NO_2$ | $CF_3$ | $-(CH_2)_3-$ | – | 121–122 |
| $CF_3$ | CN | $CF_3$ | $-(CH_2)_3-$ | – | 144–146 |
| Cl | $NO_2$ | $CF_3$ | $-(CH_2)_3-$ | – | 97–99 |
| Cl | CN | $CF_3$ | $-(CH_2)_3-$ | – | 119–120 |
| $CF_3$ | $NO_2$ | $CF_3$ | $-(CH_2)_4-$ | – | 108 |
| $CF_3$ | $NO_2$ | $CF_3$ | $-(CH_2)_7-$ | – | 84–86 |

* The chromatographic purification step was omitted as the product crystallised directly upon isolation.

All the anilines used as starting materials are known compounds. The 2-hydroxy-phenylalkanoic acids were obtained by a similar process to that described in the second part of Example 1 from the appropriate arylalkanone cyanhydrin. Those acids which are novel and which were characterised by melting point are described in the following table:-

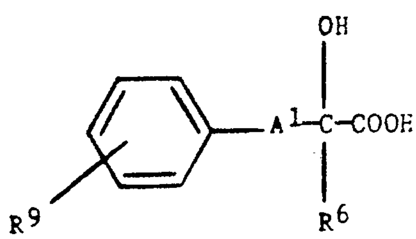

| $R^9$ | $A^1$ | $R^6$ | m.p. (°C.) |
|---|---|---|---|
| 2-Cl | $-CH_2-$ | $CF_3$ | 123-125 |
| 3-Cl | $-CH_2-$ | $CF_3$ | 106-108 |
| 4-Cl | $-CH_2-$ | $CF_3$ | 131-132 |
| 4-$CH_3$ | $-CH_2-$ | $CF_3$ | 110-112 |
| 3,4-diCl | $-CH_2$ | $CF_3$ | 108-110 |
| 2,6-diCl | $-CH_2-$ | $CF_3$ | 98-101 |
| 2-F | $-CH_2-$ | $CF_3$ | 100-102 |
| 3-F | $-CH_2-$ | $CF_3$ | 100-101 |
| 4-F | $-CH_2-$ | $CF_3$ | 108-111 |
| 4-OH | $-CH_2-$ | $CF_3$ | 176-177 |
| 4-$CH_3S$ | $-CH_2-$ | $CF_3$ | 125-126 |
| - | $-CH_2-$ | $CH_2Cl$ | 125-128 |
| - | $-CH_2-$ | 2-thienyl | 140-142 |
| - | $-CH_2CH_2-$ | $CF_3$ | 104-105 |
| - | $-(CH_2)_3-$ | $CF_3$ | 95-96 |
| - | $-(CH_2)_4-$ | $CF_3$ | 86-88 |

The arylalkanones were prepared from the appropriate Grignard reagent by the general process described in the Journal of Organic Chemistry, 1967, 32, 1316. Those which are novel and which were characterised by boiling point are described in the following table:-

| $R^9$ | $A^1$ | $R^6$ | b.p.(°C./mm.Hg.) |
|---|---|---|---|
| 2-Cl | $-CH_2-$ | $CF_3$ | 90-93/15 |
| 3-Cl | $-CH_2-$ | $CF_3$ | 87-89/15 |
| 4-Cl | $-CH_2-$ | $CF_3$ | 95-98/15 |
| 4-$CH_3$ | $-CH_2-$ | $CF_3$ | 78/15 |
| 3,4-diCl | $-CH_2-$ | $CF_3$ | 120-123/10 |
| 2,6-diCl | $-CH_2-$ | $CF_3$ | 90-95/6.5 |
| 2-F | $-CH_2-$ | $CF_3$ | 44-45/6 |
| 3-F | $-CH_2-$ | $CF_3$ | 60-62/6 |
| 4-F | $-CH_2-$ | $CF_3$ | 60-62/5 |
| 4-$CH_3S$ | $-CH_2-$ | $CF_3$ | 108-109/4 |
| - | $-CH_2CH_2-$ | $CF_3$ | 86-89/20 |
| - | $-(CH_2)_3-$ | $CF_3$ | 95-100/15 |
| - | $-(CH_2)_4-$ | $CF_3$ | 106-111/10 |
| - | $-(CH_2)_7-$ | $CF_3$ | 121-124/3 |

Example 3

A solution of sodium metaperiodate (0.6 g.) in water (10 ml.) was added to a stirred solution of 4-cyano-3-methylthio-N-(-2-hydroxy-3-phenyl-2-trifluoro-methylpropionyl)aniline (0.9 g. ) in methanol (75 ml.) and the reaction mixture was stirred at laboratory temperature for 24 hours and then filtered. The filtrate was shaken with 10% w/v aqueous sodium thiosulphate solution (25 ml.), the mixture was filtered and the filtrate was extracted three times with ethyl acetate (25 ml. each time). The combined extracts were dried over magnesium sulphate and evaporated to dryness under reduced pressure, and the residue was crystallised from toluene. There was thus obtained 4-cyano-3-methylsulphinyl-N-(2-hydroxy-3-phenyl-2-trifluoromethylpropionyl)aniline,m.p. 92-97°C.

The process described above was repeated using 4-nitro-3-trifluoromethyl-N-(2-hydroxy-3-p-methylthiophenyl-2-trifluoromethylpropionyl)aniline as starting material, and there was thus obtained 4-nitro-3-trifluoromethyl-N-(2-hydroxy-3-p-methylsulphinylphenyl-2-trifluoromethylpropionyl)aniline, m.p. 193-196°C.

The process described above was repeated using 3-chloro-4-cyano-N-(2-hydroxy-3-p-methylthiophenyl)-2-trifluoromethylpropionyl)aniline as starting material, and there was thus obtained 3-chloro-4-cyano-N-(2-hydroxy-3-p-methylsulphinylphenyl-2-trifluoromelthylpropionyl)aniline, m.p. 210-213° C.

The process described above was repeated using 3-chloro-4-methylthio-N-(2-hydroxy-3-phenyl-2-trifluoromethylpropionyl)aniline as starting material, and there was thus obtained 3-chloro-4-methylsulphinyl-N-(2-hydroxy-3-phenyl-2-trifluoromethylpropionyl)aniline,m.p. 185-187° C.

Example 4

A solution of m-chloroperbenzoic acid (1.2 g.) in methylene chloride (70 ml.) was added dropwise to a stirred solution of 3-chloro-4-cyano-N-(2-hydroxy-3-p-methylthiophenyl-2-trifluoromethylpropionyl)aniline (1.1g.)in methylene chloride (180 ml.) and the mixture was stirred at laboratory temperature for 15 hours and then shaken with 10% w/v aqueous sodium sulphite solution (45 ml.). The methylene chloride phase was separated, washed three times with saturated aqueous sodium bicarbonate solution (25 ml. ech time) and then with saturated sodium chloride solution (25 ml.), then filtered through phase-separating paper, dried and evaporated to dryness under reduced pressure. The residue was stirred with petroleum ether (b.p. 60-80° C.) and the mixture was filtered. There was thus obtained as solid residue 3-chloro-4-cyano-N-(2-hydroxy-3-p-methyl-sulphonylphenyl-2-trifluoromethylpropionyl)aniline, m.p. 204-205° C.

The process described above was repeated using the appropriate aniline as starting material and there were thus obtained the compounds described in the following table:-

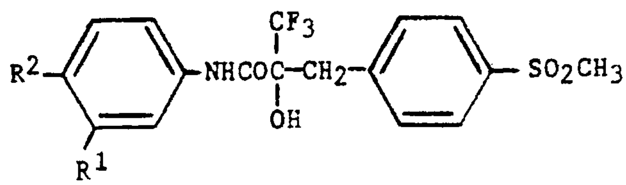

| $R^1$ | $R^2$ | m.p. (° C.) |
|---|---|---|
| $CF_3$ | $NO_2$ | 204-205 |
| $CF_3$ | CN | 220-230 |
| Cl | $NO_2$ | 197-198 |

Example 5

A solution of racemic 4-nitro-3-trifluoromethyl-N-(3-p-fluorophenyl-2-hydroxy-2-trifluoromethylpropionyl)-aniline(6.5 g.) and (-)-camphanoyl chloride (4.8 g.) in pyridine (25 ml.) was heated at 95° C. for 3 hours and then poured into water (400 ml.), and the mixture was extracted three times with ethyl acetate (100 ml. each time). The combined extracts were washed twice with saturated aqueous sodium chloride solution, dried over magnesium sulphate and evaporated to dryness under reduced pressure. The residue was dissolved in methylene chloride (15 ml.) and the solution was flash-chromatographed on silica gel (Merck 9385; 450 g.) using methylene chloride as eluant. There were thus obtained the two diastereoisomers of 4-nitro-3-trifluoromethyl-N-[2-(-)-camphanoyloxy-3-p-fluorophenyl-3-trifluoromethylpropionyl]aniline, the less polar isomer having m.p. 142° C. and the more polar isomer having m.p.65-72° C.

A mixture of a solution of the less polar isomer (3.0g.) in methanol (20 ml.) and a solution of sodium hydroxide (0.2 g.) in water (3.5 ml.) was stirred at laboratory temperature for 30 minutes and the methanol was then removed by evaporation under reduced pressure. Water (40 ml.) was added and the mixture was extracted three times with ethyl acetate (25 ml. each time). The combined extracts were successively washed (25 ml. portions each time) twice with aqueous 2N-hydrochloric acid, twice with water and once with saturated aqueous sodium chloride solution, dried over magnesium sulphate and evaporated to dryness. The residue was stirred with petroleum ether (b.p. 60-80° C.) and the mixture was filtered. There was thus obtained as solid residue (+)-4-nitro-3-trifluoromethyl-N-(3-p-fluorophenyl-2-hydroxy-2-trifluoromethylpropionyl)aniline, m.p. 118-120° C., $[\alpha]^{25}$ = + 197.2 (C, 1%in methanol).

The process described in the preceding paragraph was repeated using the more polar isomer of the camphanoyl ester, and the hydrolysis product was crystallised from a 10:1 v/v mixture of petroleum ether (b.p. 60-80° C.) and toluene. There was thus obtained (-)-4-nitro-3-trifluoromethyl-N-(3-p-fluorophenyl-2-hydroxy-2-trifluoromethylpropionyl)aniline, m.p. 105-107° C., $[\alpha]^{25}$ = -195.2 (C, 1% methanol).

Example 6

A solution of 3,4-dichloro-N-(2,3-epoxy-2-methylpropionyl)aniline (1.23 g.) in diethyl ether (15 ml.) was added dropwise to a solution of 2-thienyllithium [prepared by the addition of 6.25 ml. of a 1.6 molar solution of butyllithium in hexane to a solution of thiophene (1.15 g.) in diethyl ether (15 ml.)] at such a rate that the temperature of the mixture did not rise above 30° C. The mixture was stirred at laboratory temperature for 1.5 hours and poured into water (50 ml.). The organic layer was separated and the aqueous layer was extracted twice with diethyl ether (25 ml. each time). The combined extracts were washed with water and with a saturated aqueous sodium chloride solution, dried over magnesium sulphate and evaporated to dryness under reduced pressure. The residual oil was purified by chromatography on silica gel using methylene chloride as eluant. There was thus obtained 3,4-dichloro-N-[2-hydroxy-2-methyl-3-(2-thienyl)-propionyl]aniline,m.p. 68-71° C.

The 3,4-dichloro-N-(2,3-epoxy-2-methylpropionyl)aniline used as starting material was prepared by the reaction of 3,4-dichloro-N-methacryloylaniline (prepared as described in the Journal of Organic Chemistry, 1963, 28, 2915) and m-chloroperbenzoic acid using the method described in the Journal of the Chemical Society, Chemical Communications, 1972, 64.

## Claims

**1.** An acylanilide of the formula:-

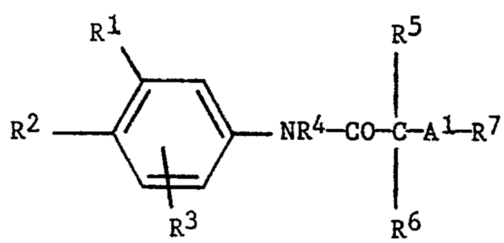

wherein $R^1$ is cyano, carbamoyl, nitro, fluoro, chloro, bromo, iodo or hydrogen, or alkyl, alkoxy, alkanoyl, alkylthio, alkylsulphinyl, alkylsulphonyl, perfluoroalkyl, perfluoroalkylthio, perfluoroalkylsulphinyl or perfluoroalkylsulphonyl each of up to 4 carbon atoms, or phenylthio, phenylsulphinyl or phenylsulphonyl;
wherein $R^2$ is cyano, carbamoyl, nitro, fluoro, chloro, bromo or iodo, or alkanoyl, alkylthio, alkylsulphinyl, alkylsulphonyl, perfluoroalkyl, perfluoroalkylthio, perfluoroalkylsulphinyl or perfluoroalkylsulphonyl each of up to 4 carbon atoms, or phenylthio, phenylsulphinyl or phenylsulphonyl;
wherein $R^3$ is hydrogen or halogen;
wherein $R^4$ is hydrogen or alkyl of up to 4 carbon atoms, or is joined to $R^5$ as stated below;
wherein $R^5$ is hydroxy or alkoxy or acyloxy each of up to 15 carbon atoms, or is joined to $R^4$ to form an oxycarbonyl group such that together with the -N-CO-C- part of the molecule it forms an oxazolidinedione group;
wherein $R^6$ is alkyl or halogenoalkyl each of up to 4 carbon atoms;
wherein $A^1$ is straight-chain alkylene of up to 10 carbon atoms, or alkenylene or alkynylene each of 2 to 10 carbon atoms;
and wherein $R^7$ is selected from phenyl which bears one, two or three substituents selected from hydrogen, halogen, nitro, hydroxy, carboxy, carbamoyl and cyano, alkyl, alkoxy, alkanoyl, alkylthio, alkylsulphinyl, alkylsulphonyl, perfluoroalkyl, perfluoroalkylthio, perfluoroalkylsulphinyl, perfluoroalkylsulphonyl, alkoxycarbonyl and N-alkylcarbamoyl each of up to 4 carbon atoms, phenyl, phenylthio, phenylsulphinyl and phenylsulphonyl, naphthyl and 5- or 6- membered saturated or unsaturated heterocyclic which contains one, two or three heteroatoms selected from oxygen, nitrogen and sulphur, which heterocyclic may be a single ring or may be fused to a benzo-ring, and which heterocyclic is unsubstituted or bears one or two halogen, cyano or amino, alkyl, alkoxy, alkylthio, alkylsulphinyl or alkylsulphonyl each of up to 4 carbon atoms, oxy or hydroxy substituents, or which if sufficiently saturated may bear one or two oxo substituents.

2. An acylanilide as claimed in claim 1, wherein $R^1$ and $R^2$, which may be the same or different, each is cyano, nitro, trifluoromethyl, methylthio, methylsulphinyl, methylsulphonyl or chloro, $R^3$ and $R^4$ are both hydrogen, $R^5$ is hydroxy, $R^6$ is methyl or trifluoromethyl, $A^1$ is methylene, ethylene, trimethylene or tetramethylene and $R^7$ is phenyl which is unsubstituted or which bears one fluoro, chloro, hydroxy, methyl, methylthio, methylsulphinyl or methylsulphonyl substitutent.

3. An acylanilide as claimed in claim 1, wherein $R^1$ is cyano, fluoro, hydrogen, ethoxy, methylsulphonyl or trifluoromethyl, wherein $R^2$ is cyano or nitro, wherein $R^3$ and $R^4$ are both hydrogen and $R^5$ is hydroxy, wherein $R^6$ is trifluoromethyl, wherein $A^1$ is methylene, ethylene, trimethylene or tetramethylene and wherein $R^7$ is phenyl which is unsubstituted or bears one substituent selected from fluoro, chloro and methyl.

4. An acylanilide selected from the group of compounds of the formula:-

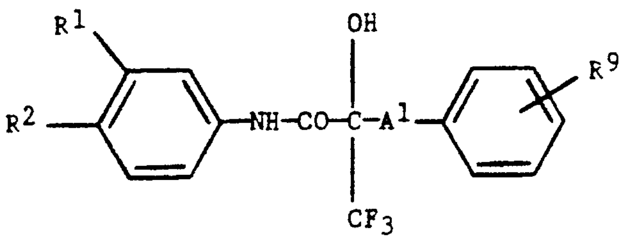

wherein the specific values of $R^1$, $R^2$, $A^1$ and $R^9$ are shown in the following table:-

| $R^1$ | $R^2$ | $A^1$ | $R^9$ |
|---|---|---|---|
| $CF_3$ | CN | $-CH_2-$ | H |
| $CF_3$ | $NO_2$ | $-CH_2-$ | H |
| $CF_3$ | $NO_2$ | $-CH_2-$ | 4-$CH_3$ |
| $CF_3$ | $NO_2$ | $-CH_2-$ | 2-Cl |
| $CF_3$ | $NO_2$ | $-CH_2-$ | 3-Cl |
| $CF_3$ | $NO_2$ | $-CH_2-$ | 4-Cl |
| $CF_3$ | $NO_2$ | $-CH_2-$ | 2-F |
| $CF_3$ | $NO_2$ | $-CH_2-$ | 3-F |
| Cl | $NO_2$ | $-CH_2-$ | H |
| Cl | CN | $-CH_2-$ | 2-Cl |
| Cl | CN | $-CH_2-$ | 3-Cl |
| Cl | CN | $-CH_2-$ | 4-Cl |
| Cl | CN | $-CH_2-$ | 3-F |
| $C_2H_5O$ | $NO_2$ | $-CH_2-$ | H |
| $CH_3SO_2$ | $NO_2$ | $-CH_2-$ | H |
| $CF_3$ | $NO_2$ | $-CH_2CH_2-$ | H |
| $CF_3$ | $NO_2$ | $-(CH_2)_4-$ | H |
| $CF_3$ | $NO_2$ | $-CH_2-$ | 4-F |

5. An acylanilide selected from the group of compounds of the formula:-

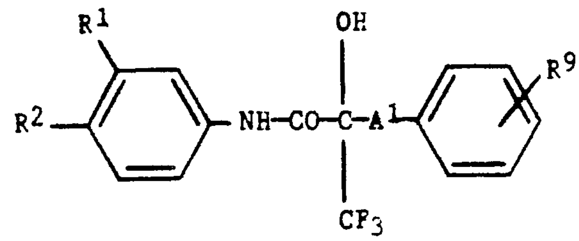

wherein the specific values of $R^1$, $R^2$, $A^1$ and $R^9$ are shown in the following table:-

| $R^1$ | $R^2$ | $A^1$ | $R^9$ |
|---|---|---|---|
| Cl | CN | $-CH_2-$ | H |
| $CF_3$ | CN | $-CH_2-$ | H |
| $CF_3$ | CN | $-CH_2-$ | 2-Cl |
| $CF_3$ | CN | $-CH_2-$ | 2-F |
| $CF_3$ | CN | $-CH_2-$ | 3-F |
| $CF_3$ | CN | $-CH_2-$ | 4-F |
| $CF_3$ | $NO_2$ | $-CH_2-$ | H |
| $CF_3$ | $NO_2$ | $-CH_2-$ | 4-Cl |
| $CF_3$ | $NO_2$ | $-CH_2-$ | 2-F |
| $CF_3$ | $NO_2$ | $-CH_2-$ | 3-F |
| Cl | $NO_2$ | $-CH_2-$ | H |
| Cl | CN | $-CH_2-$ | 2-Cl |
| Cl | CN | $-CH_2-$ | 4-Cl |
| Cl | CN | $-CH_2-$ | 2-F |
| Cl | CN | $-CH_2-$ | 3-F |
| Cl | CN | $-CH_2-$ | 4-F |
| F | CN | $-CH_2-$ | H |
| F | CN | $-CH_2-$ | 4-F |
| CN | CN | $-CH_2-$ | H |
| H | CN | $-CH_2-$ | H |
| H | $NO_2$ | $-CH_2-$ | H |
| H | $NO_2$ | $-CH_2-$ | 2-Cl |
| $CF_3$ | $NO_2$ | $-(CH_2)_3$ | H |
| $CF_3$ | $NO_2$ | $-CH_2-$ | 4-F |

6. The compound 3-chloro-4-cyano-N-(2-hydroxy-3-p-methanesulphonylphenyl-2-trifluoromethylpropionyl)-aniline;
3-chloro-4-cyano-N-(3-p-fluorophenyl-2-hydroxy-2-trifluoromethylpropionyl)aniline;
4-cyano-3-trifluoromethyl-N-(3-p-chlorophenyl-2-hydroxy-2-trifluoromethylpropionyl)aniline;
4-cyano-3-trifluoromethyl-N-(2-hydroxy-3-phenyl-2-trifluoromethylpropionyl)aniline;
4-nitro-3-trifluoromethyl-N-(2-hydroxy-3-phenyl-2-trifluoromethylpropionyl)aniline;
4-nitro-3-trifluoromethyl-N-(3-p-chlorophenyl-2-hydroxy-2-trifluoromethylpropionyl)aniline;
4-nitro-3-trifluoromethyl-N-(3-p-fluorophenyl-2-hydroxy-2-trifluoromethylpropionyl)aniline;
4-nitro-N-(3-p-fluorophenyl-2-hydroxy-2-trifluoromethylpropionyl)aniline;
4-cyano-N-(2-hydroxy-2-trifluoromethyl-4-phenylbutyryl)aniline;
4-nitro-3-trifluoromethyl-N-(3-o-chlorophenyl-2-hydroxy-2-trifluoromethylpropionyl)aniline;
4-nitro-3-trifluoromethyl-N-(3-m-fluorophenyl-2-hydroxy-2-trifluoromethylpropionyl)anilineor
4-cyano-3-trifluoromethyl-N-(3-p-fluorophenyl-2-hydroxy-2-trifluoromethylpropionyl)aniline.

7. A process for the manufacture of an acylanilide claimed in claim 1 which comprises:-
(a) the reaction of an amine of the formula

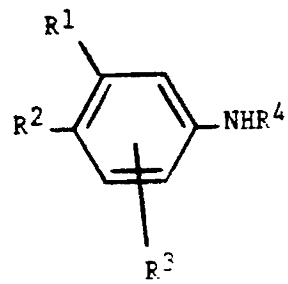

wherein $R^1$, $R^2$, $R^3$ and $R^4$ have the meanings stated in claim 1, with an acid of the formula:-

$HO_2C\text{-}CR^5R^6\text{-}A^1\text{-}R^7$

wherein $R^5$, $R^6$, $R^7$ and $A^1$ have the meanings stated in claim 1 or with a reactive derivative of said acid;

(b) for the manufacture of an acylanilide of the invention wherein $R^4$ and $R^5$ are joined together to form a carbonyl-oxy group, that is, an oxazolidinedione, the reaction of an isocyanate of the formula:-

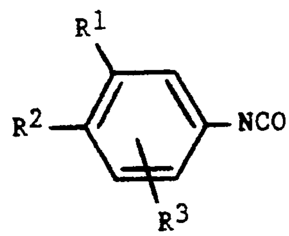

wherein $R^1$, $R^2$ and $R^3$ have the meanings stated in claim 1, with an ester of the formula:-

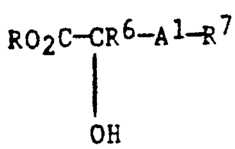

wherein $R^6$, $R^7$ and $A^1$ have the meanings stated in claim 1 and wherein R is alkyl of up to 6 carbon atoms;

(c) for the manufacture of an acylanilide of the invention wherein $R^7$ is heterocylic and $A^1$ is methylene, the reaction of an epoxide of the formula:-

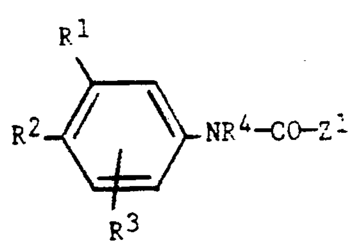

wherein $R^1$, $R^2$, $R^3$ and $R^4$ have the meanings stated in claim 1 and wherein $Z^1$ has the formula:-

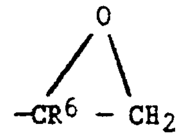

wherein $R^6$ has the meaning stated in claim 1, with a heterocycle or a reactive derivative thereof of the formula $R^7$-M wherein $R^7$ has the meaning stated immediately above and M is a metal radical;
(d) for the manufacture of an acylanilide of the invention wherein $R^5$ is hydroxy, the hydrolysis of the corresponding acylanilide wherein $R^5$ is acyloxy;
(e) for the manufacture of an acylanilide of the invention wherein $R^5$ is hydroxy and $R^4$ is hydrogen, the hydrolysis of the corresponding oxazolidinedione;
(f) for the manufacture of an acylanilide of the invention wherein $R^4$ is alkyl, the alkylation of the corresponding acylanilide wherein $R^4$ is hydrogen;
(g) for the manufacture of an acylanilide of the invention wherein $R^5$ is acyloxy, the acylation of the corresponding acylanilide wherein $R^5$ is hydroxy;
(h) for the manufacture of an acylanilide of the invention wherein $R^4$ and $R^5$ are joined together to form a carbonyl-oxy group, the reaction of the corresponding acylanilide wherein $R^4$ is hydrogen and $R^5$ is hydroxy with phosgene ($COCl_2$);
(i) for the manufacture of an acylanilide of the invention wherein one or more of $R^1$, $R^2$ and a substituent in the phenyl or heterocyclic group $R^7$ is alkylsulphinyl, perfluoroalkylsulphinyl or phenylsulphinyl, or is alkylsulphonyl, perfluoroalkylsulphonyl or phenylsulphonyl, the oxidation of the corresponding acylanilide wherein one or more of $R^1$, $R^2$ and a substituent in the phenyl or heterocyclic group $R^7$ is alkylthio, perfluoroalkylthio or phenylthio, respectively; or
(j) for the separation into its optical isomers of an acylanilide of the invention wherein $R^5$ is hydroxy, the formation of an ester of the hydroxy group $R^5$ with an optically-active acid, the separation of the diastereoisomeric esters thus obtained, by fractional crystallisation or by flash-chromatography, and then the hydrolysis of each separate ester to the alcohol.

**8.** A pharmaceutical composition comprising an acylanilide as claimed in claim 1, together with a pharmaceutically acceptable diluent or carrier; the composition optionally containing one or more drugs selected from anti-oestrogens, progestins, inhibitors of gonodotrophin secretion, cytotoxic agents, antibiotics and anti-inflammatory agents.

**9.** The use of an acylanilide as claimed in claim 1 for the manufacture of a medicament for producing an antiandrogenic effect in a warm-blooded animal.

**10.** The use of an acylanilide as claimed in claim 4 for the manufacture of a medicament for producing a progestational effect in a warm-blooded animal.

**11.** The use of an acylanilide as claimed in claim 5 for the manufacture of a medicament for producing an antiprogestational effect in a warm-blooded animal.

## Revendications

**1.** Acylanilide de formule :

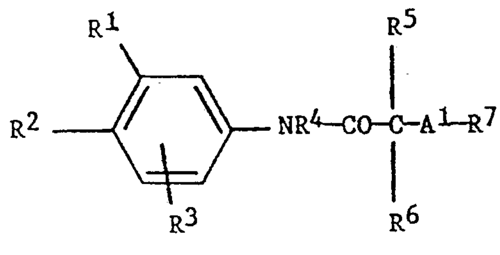

dans laquelle
$R^1$ est un groupe cyano, carbamoyle, nitro, fluoro, chloro, bromo, iodo ou hydrogène, ou un groupe

alkyle, alkoxy, alcanoyle, alkylthio, alkylsulfinyle, alkylsulfonyle, perfluoralkyle, perfluoralkylthio, perfluoralkylsulfinyle ou perfluoralkylsulfonyle ayant chacun jusqu'à 4 atomes de carbone, ou un groupe phénylthio, phénylsulfinyle ou phénylsulfonyle ;

$R^2$ est un groupe cyano, carbamoyle, nitro, fluoro, chloro, bromo ou iodo ou un groupe alcanoyle, alkylthio, alkylsulfinyle, alkylsulfonyle, perfluoralkyle, perfluoralkylthio, perfluoralkylsulfinyle ou perfluoralkylsulfonyle ayant chacun jusqu'à 4 atomes de carbone, ou un groupe phénylthio, phénylsulfinyle ou phénylsulfonyle ;

$R^3$ est l'hydrogène ou un halogène ;

$R^4$ est l'hydrogène ou un groupe alkyle ayant jusqu'à 4 atomes de carbone, ou est associé à $R^5$ comme indiqué ci-dessous ;

$R^5$ est un groupe hydroxy, ou un groupe alkoxy ou acyloxy ayant chacun jusqu'à 15 atomes de carbone, ou s'associe à $R^4$ pour former un groupe oxycarbonyle de façon telle qu'il forme conjointement avec la partie -N-CO-C- de la molécule un groupe oxazolidinedione ;

$R^6$ est un groupe alkyle ou un groupe halogénalkyle ayant chacun jusqu'à 4 atomes de carbone ;

$A^1$ est un groupe alkylène à chaîne droite ayant jusqu'à 10 atomes de carbone, ou un groupe alcénylène ou alcynylène ayant chacun 2 à 10 atomes de carbone ; et

$R^7$ est choisi entre un groupe phényle qui porte un, deux ou trois substituants choisis entre l'hydrogène, un halogène, un radical nitro, hydroxy, carboxy, carbamoyle et cyano, des groupes alkyle, alkoxy, alcanoyle, alkylthio, alkylsulfinyle, alkylsulfonyle, perfluoralkyle, perfluoralkylthio, perfluoralkylsulfinyle, perfluoralkylsulfonyle, alkoxycarbonyle et N-alkylcarbamoyle ayant chacun jusqu'à 4 atomes de carbone, phényle, phénylthio, phénylsulfinyle et phénylsulfonyle, naphtyle, et un groupe hétérocyclique saturé ou non saturé pentagonal ou hexagonal qui contient un, deux ou trois hétéroatomes choisis entre les atomes d'oxygène, d'azote et de soufre, ce groupe hétérocyclique pouvant être un simple noyau ou pouvant être condensé à un noyau benzénique, et ce groupe hétérocyclique étant non substitué ou portant un ou deux substituants halogéno, cyano ou amino, alkyle, alkoxy, alkylthio, alkylsulfinyle ou alkylsulfonyle ayant chacun jusqu'à 4 atomes de carbone, oxy ou hydroxy, ou qui peut porter un ou deux substituants oxo s'il est suffisamment saturé.

2. Acylanilide suivant la revendication 1, dans lequel $R^1$ et $R^2$, qui peuvent être identiques ou différents, représentent chacun un groupe cyano, nitro, trifluorométhyle, méthylthio, méthylsulfinyle, méthylsulfonyle ou chloro, $R^3$ et $R^4$ sont tous deux de l'hydrogène, $R^5$ est un groupe hydroxy, $R^6$ est un groupe méthyle ou trifluorométhyle, $A^1$ est un groupe méthylène, éthylène, triméthylène ou tétraméthylène, et $R^7$ est un groupe phényle qui est non substitué ou qui porte un substituant fluoro, chloro, hydroxy, méthyle, méthylthio, méthylsulfinyle ou méthylsulfonyle.

3. Acylanilide suivant la revendication 1, dans lequel $R^1$ est un groupe cyano, fluoro, l'hydrogène, un groupe éthoxy, méthylsulfonyle ou trifluorométhyle, $R^2$ est un groupe cyano ou nitro, $R^3$ et $R^4$ représentent tous deux de l'hydrogène et $R^5$ est un groupe hydroxy, $R^6$ est un groupe trifluorométhyle, $A^1$ est un groupe méthylène, éthylène, triméthylène ou tétraméthylène, et $R^7$ est un groupe phényle qui est non substitué ou qui porte un substituant choisi entre les substituants fluoro, chloro et méthyle.

4. Acylanilide choisi dans le groupe de composés de formule :

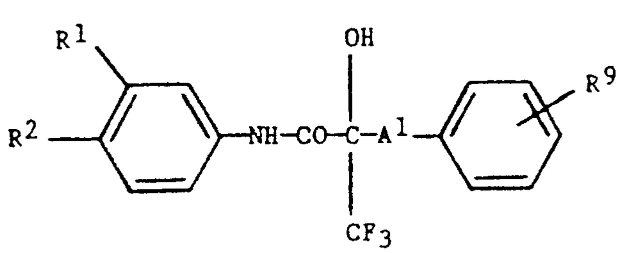

dans laquelle les valeurs particulières de $R^1$, $R^2$, $A^1$ et $R^9$ sont représentées sur le tableau suivant :

| $R^1$ | $R^2$ | $A^1$ | $R^9$ |
|---|---|---|---|
| $CF_3$ | CN | $-CH_2-$ | H |
| $CF_3$ | $NO_2$ | $-CH_2-$ | H |
| $CF_3$ | $NO_2$ | $-CH_2-$ | $4-CH_3$ |
| $CF_3$ | $NO_2$ | $-CH_2-$ | 2-Cl |
| $CF_3$ | $NO_2$ | $-CH_2-$ | 3-Cl |
| $CF_3$ | $NO_2$ | $-CH_2-$ | 4-Cl |
| $CF_3$ | $NO_2$ | $-CH_2-$ | 2-F |
| $CF_3$ | $NO_2$ | $-CH_2-$ | 3-F |
| Cl | $NO_2$ | $-CH_2-$ | H |
| Cl | CN | $-CH_2-$ | 2-Cl |
| Cl | CN | $-CH_2-$ | 3-Cl |
| Cl | CN | $-CH_2-$ | 4-Cl |
| Cl | CN | $-CH_2-$ | 3-F |
| $C_2H_5O$ | $NO_2$ | $-CH_2-$ | H |
| $CH_3SO_2$ | $NO_2$ | $-CH_2-$ | H |
| $CF_3$ | $NO_2$ | $-CH_2CH_2-$ | H |
| $CF_3$ | $NO_2$ | $-(CH_2)_4-$ | H |
| $CF_3$ | $NO_2$ | $-CH_2-$ | 4-F |

**5.** Acylanilide choisi dans le groupe de composés de formule :

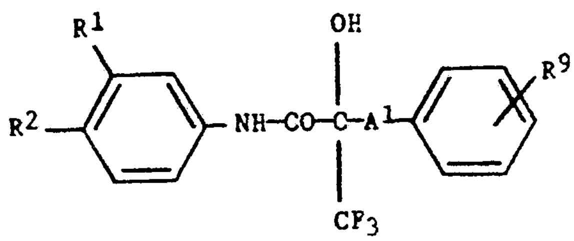

dont les valeurs particulières de $R^1$, $R^2$, $A^1$ et $R^9$ sont représentées sur le tableau suivant :

| $R^1$ | $R^2$ | $A^1$ | $R^9$ |
|---|---|---|---|
| Cl | CN | $-CH_2-$ | H |
| $CF_3$ | CN | $-CH_2-$ | H |
| $CF_3$ | CN | $-CH_2-$ | 2-Cl |
| $CF_3$ | CN | $-CH_2-$ | 2-F |
| $CF_3$ | CN | $-CH_2-$ | 3-F |
| $CF_3$ | CN | $-CH_2-$ | 4-F |
| $CF_3$ | $NO_2$ | $-CH_2-$ | H |
| $CF_3$ | $NO_2$ | $-CH_2-$ | 4-Cl |
| $CF_3$ | $NO_2$ | $-CH_2-$ | 2-F |
| $CF_3$ | $NO_2$ | $-CH_2-$ | 3-F |
| Cl | $NO_2$ | $-CH_2-$ | H |
| Cl | CN | $-CH_2-$ | 2-Cl |
| Cl | CN | $-CH_2-$ | 4-Cl |
| Cl | CN | $-CH_2-$ | 2-F |
| Cl | CN | $-CH_2-$ | 3-F |
| Cl | CN | $-CH_2-$ | 4-F |
| F | CN | $-CH_2-$ | H |
| F | CN | $-CH_2-$ | 4-F |
| CN | CN | $-CH_2-$ | H |
| H | CN | $-CH_2-$ | H |
| H | $NO_2$ | $-CH_2-$ | H |
| H | $NO_2$ | $-CH_2-$ | 2-Cl |
| $CF_3$ | $NO_2$ | $-(CH_2)_3$ | H |
| $CF_3$ | $NO_2$ | $-CH_2-$ | 4-F |

6. La 3-chloro-4-cyano-N-(2-hydroxy-3-p-méthanesulfonylphényl-2-trifluorométhylpropionyl)aniline ;
la 3-chloro-4-cyano-N-(3-p-fluorophényl-2-hydroxy-2-trifluorométhylpropionyl)aniline ;
la 4-cyano-3-trifluorométhyl-N-(3-p-chlorophényl-2-hydroxy-2-trifluorométhylpropionyl)aniline ;
la 4-cyano-3-trifluorométhyl-N-(2-hydroxy-3-phényl-2-trifluorométhylpropionyl)aniline ;
la 4-nitro-3-trifluorométhyl-N-(2-hydroxy-3-phényl-2-trifluorométhylpropionyl)aniline ;
la 4-nitro-3-trifluorométhyl-N-(3-p-chlorophényl-2-hydroxy-2-trifluorométhylpropionyl)aniline ;
la 4-nitro-3-trifluorométhyl-N-(3-p-fluorophényl-2-hydroxy-2-trifluorométhylpropionyl)aniline ;
la 4-nitro-N-(3-p-fluorophényl-2-hydroxy-2-trifluorométhylpropionyl)aniline ;
la 4-cyano-N-(2-hydroxy-2-trifluorométhyl-4-phénylbutyryl)aniline ;
la 4-nitro-3-trifluorométhyl-N-(3-o-chlorophényl-2-hydroxy-2-trifluorométhylpropionyl)aniline ;
la 4-nitro-3-trifluorométhyl-N-(3-m-fluorophényl-2-hydroxy-2-trifluorométhylpropionyl)aniline ou
la 4-cyano-3-trifluorométhyl-N-(3-P-fluorophényl-2-hydroxy-2-trifluorométhylpropionyl)aniline.

7. Procédé de préparation d'un acylanilide suivant la revendication 1, qui comprend :
(a) la réaction d'une amine de formule

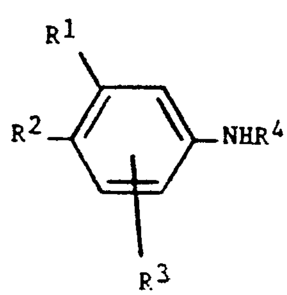

dans laquelle $R^1$, $R^2$, $R^3$ et $R^4$ ont les définitions indiquées dans la revendication 1, avec un acide de formule :

$HO_2C-CR^5R^6-A^1-R^7$

dans laquelle $R^5$, $R^6$, $R^7$ et $A^1$ ont les définitions indiquées dans la revendication 1 ou avec un dérivé réactif dudit acide ;

(b) pour la préparation d'un acylanilide de l'invention dans lequel $R^4$ et $R^5$ s'associent pour former un groupe carbonyloxy, c'est-à-dire une oxazolidinedione, la réaction d'un isocyanate de formule :

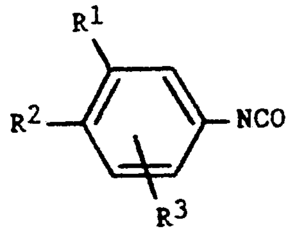

dans laquelle $R^1$, $R^2$ et $R^3$ ont les définitions indiquées dans la revendication 1, avec un ester de formule :

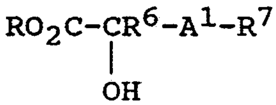

dans laquelle $R^6$, $R^7$ et $A^1$ ont les définitions indiquées dans la revendication 1 et R est un groupe alkyle ayant jusqu'à 6 atomes de carbone ;

(c) pour la préparation d'un acylanilide de l'invention dans lequel $R^7$ est un groupe hétérocyclique et $A^1$ est un groupe méthylène, la réaction d'un époxyde de formule :

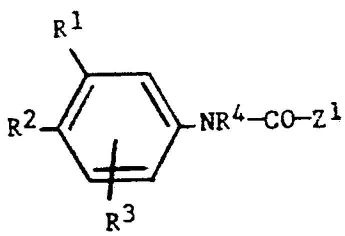

dans laquelle $R^1$, $R^2$, $R^3$ et $R^4$ ont les définitions indiquées dans la revendication 1 et $Z^1$ répond à la formule :

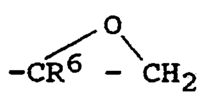

où $R^6$ a la définition indiquée dans la revendication 1, avec un hétérocycle ou un dérivé réactif d'un hétérocycle de formule $R^7$-M dans laquelle $R^7$ a la définition qui vient d'être donnée ci-dessus et M est un radical métallique ;

(d) pour la préparation d'un acylanilide de l'invention dans lequel $R^5$ est un groupe hydroxy, l'hydrolyse de l'acylanilide correspondant dans lequel $R^5$ est un groupe acyloxy ;

(e) pour la préparation d'un acylanilide de l'invention dans lequel $R^5$ est un groupe hydroxy et $R^4$ est l'hydrogène, l'hydrolyse de l'oxazolidinedione correspondante.

(f) pour la préparation d'un acylanilide de l'invention dans lequel $R^4$ est un groupe alkyle, l'alkylation de l'acylanilide correspondant dans lequel $R^4$ est l'hydrogène ;

(g) pour la préparation d'un acylanilide de l'invention dans lequel $R^5$ est un groupe acyloxy, l'acylation de l'acylanilide correspondant dans lequel $R^5$ est un groupe hydroxy ;

(h) pour la préparation d'un acylanilide de l'invention dans lequel $R^4$ et $R^5$ s'associent pour former

un groupe carbonyloxy, la réaction de l'acylanilide correspondant dans lequel $R^4$ est l'hydrogène et $R^5$ est un groupe hydroxy avec le phosgène ($COCl_2$) ;
(i) pour la préparation d'un acylanilide de l'invention dans lequel un ou plusieurs de $R^1$, $R^2$ et un substituant du groupe phényle ou hétérocyclique $R^7$ sont un groupe alkylsulfinyle, perfluoroalkylsulfinyle ou phénylsulfinyle ou est un groupe alkylsulfonyle, perfluoroalkylsulfonyle ou phénylsulfonyle, l'oxydation de l'acylanilide correspondant dans lequel un ou plusieurs de $R^1$, $R^2$ et un substituant du groupe phényle ou hétérocyclique $R^7$ sont un radical alkylthio, perfluoroalkylthio, ou phénylthio, respectivement ; ou
(j) pour le fractionnement en ses isomères optiques d'un acylanilide de l'invention dans lequel $R^5$ est un groupe hydroxy, la formation d'un ester du groupe hydroxy $R^5$ avec un acide optiquement actif, la séparation des esters diastéréo-isomériques ainsi obtenus par cristallisation fractionnée ou par chromatographie instantanée, puis l'hydrolyse en l'alcool de chaque ester séparé.

**8.** Composition pharmaceutique comprenant un acylanilide suivant la revendication 1, en association avec un diluant ou support acceptable du point de vue pharmaceutique ; la composition contenant facultativement un ou plusieurs médicaments choisis entre des anti-oestrogènes, des progestines, des inhibiteurs de sécrétion de gonadotrophine, des agents cytotoxiques, des antibiotiques et des agents anti-inflammatoires.

**9.** Utilisation d'un acylanilide suivant la revendication 1 pour la préparation d'un médicament destiné à produire un effet anti-endrogénique chez un animal à sang chaud.

**10.** Utilisation d'un acylanilide suivant la revendication 4, pour la préparation d'un médicament destiné à produire un effet progestationnel chez un animal à sang chaud.

**11.** Utilisation d'un acylanilide suivant la revendication 5 pour la préparation d'un médicament destiné à produire un effet anti-progestationnel chez un animal à sang chaud.

**Patentansprüche**

**1.** Acylanilid der Formel

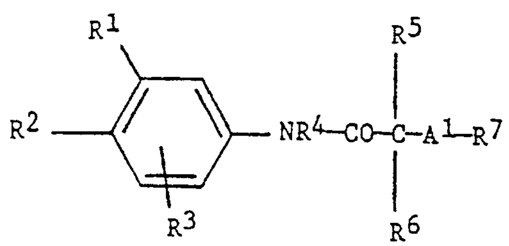

worin

$R^1$ für Cyano, Carbamoyl, Nitro, Fluoro, Chloro, Bromo, Jodo oder Wasserstoff oder für Alkyl, Alkoxy, Alkanoyl, Alkylthio, Alkylsulfinyl, Alkylsulfonyl, Perfluoroalkyl, Perfluoroalkylthio, Perfluoroalkylsulfinyl oder Perfluoroalkylsulfonyl mit jeweils bis zu 4 Kohlenstoffatomen oder für Phenylthio, Phenylsulfinyl oder Phenylsulfonyl steht;

$R^2$ für Cyano, Carbamoyl, Nitro, Fluoro, Chloro, Bromo oder Jodo oder für Alkanoyl, Alkylthio, Alkylsulfinyl, Alkylsulfonyl, Perfluoroalkyl, Perfluoroalkylthio, Perfluoroalkylsulfinyl oder Perfluoroalkylsulfonyl mit jeweils bis zu 4 Kohlenstoffatomen oder für Phenylthio, Phenylsulfinyl oder Phenylsulfonyl steht;

$R^3$ für Wasserstoff oder Halogen steht;

$R^4$ für Wasserstoff oder Alkyl mit bis zu 4 Kohlenstoffatomen steht oder mit $R^5$, wie weiter angegeben, verbunden ist;

$R^5$ für Hydroxy oder für Alkoxy oder Acyloxy mit jeweils bis zu 15 Kohlenstoffatomen steht oder mit $R^4$ unter Bildung einer Oxycarbonyl-Gruppe verbunden ist, so daß zusammen mit dem -N-CO-C- Teil des Moleküls eine Oxazolidindion-Gruppe gebildet wird.

$R^6$ für Alkyl oder Halogenoalkyl mit jeweils bis zu 4 Kohlenstoffatomen steht;

$A^1$ für gerades Alkylen mit bis zu 10 Kohlenstoffatomen oder für Alkenylen oder Alkinylen mit jeweils 2 bis 10 Kohlenstoffatomen steht; und

$R^7$ für Phenyl, das einen, zwei oder drei aus Wasserstoff, Halogen, Nitro, Hydroxy, Carboxy, Carbamoyl und Cyano ausgewählte Substituenten trägt, für Alkyl, Alkoxy, Alkanoyl, Alkylthio, Alkylsulfinyl, Alkylsulfonyl, Perfluoroalkyl, Perfluoroalkylthio, Perfluoroalkylsulfinyl, Perfluoroalkylsulfonyl, Alkoxycarbonyl oder N-Alkylcarbamoyl mit jeweils bis zu 4 Kohlenstoffatomen, für Phenyl, Phenylthio, Phenylsulfinyl oder Phenylsulfonyl, für Naphthyl oder für einen 5- oder 6gliedrigen gesättigten oder ungesättigten Heterocyclus, der ein, zwei oder drei aus Sauerstoff, Stickstoff und Schwefel ausgewählter Heteroatome enthält, steht, wobei der Heterocyclus ein einzelner Ring oder an einen Benzol-Ring kondensiert sein kann und wobei der Heterocyclus unsubstituiert ist oder einen oder zwei Substituenten trägt, die ausgewählt sind aus Halogeno, Cyano oder Amino, aus Alkyl, Alkoxy, Alkylthio, Alkylsulfinyl oder Alkylsulfonyl mit jeweis bis zu 4 Kohlenstoffatomen oder aus Oxy oder Hydroxy, oder,sofern er ausreichend gesättigt ist, einen oder zwei Oxo-Substituenten tragen kann.

2. Acylanilid nach Anspruch 1, worin $R^1$ und $R^2$, welche gleich oder verschieden sein können, jeweils für Cyano, Nitro, Trifluoromethyl, Methylthio, Methylsulfinyl, Methylsulfonyl oder Chloro stehen, $R^3$ und $R^4$ beide für Wasserstoff stehen, $R^5$ für Hydroxy steht, $R^6$ für Methyl oder Trifluoromethyl steht, $A^1$ für Methylen, Ethylen, Trimethylen oder Tetramethylen steht und $R^7$ für Phenyl steht, das unsubstituiert ist oder einen Fluoro-, Chloro-, Hydroxy-, Methyl-, Methylthio-, Methylsulfinyl- oder Methylsulfonyl-Substituenten trägt.

3. Acylanilid nach Anspruch 1, worin $R^1$ für Cyano, Fluoro, Wasserstoff, Ethoxy, Methylsulfonyl oder Trifluoromethyl steht, $R^2$ für Cyano oder Nitro steht, $R^3$ und $R^4$ beide für Wasserstoff stehen, $R^5$ für Hydroxy steht, $R^6$ für Trifluoromethyl steht, $A^1$ für Methylen, Ethylen, Trimethylen oder Tetramethylen steht und $R^7$ für Phenyl steht, das unsubstituiert ist oder einen aus Fluoro, Chloro und Methyl ausgewählten Substituenten trägt.

4. Acylanilid, welches ausgewählt ist aus Verbindungen der Formel

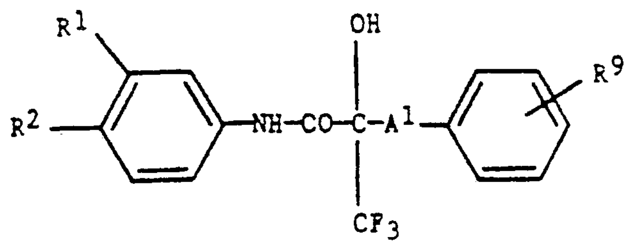

wobei die einzelnen Substituenten $R^1$, $R^2$, $A^1$ und $R^9$ in der folgenden Tabelle angegeben sind:

| $R^1$ | $R^2$ | $A^1$ | $R^9$ |
|---|---|---|---|
| $CF_3$ | CN | $-CH_2-$ | H |
| $CF_3$ | $NO_2$ | $-CH_2-$ | H |
| $CF_3$ | $NO_2$ | $-CH_2-$ | $4-CH_3$ |
| $CF_3$ | $NO_2$ | $-CH_2-$ | 2-Cl |
| $CF_3$ | $NO_2$ | $-CH_2-$ | 3-Cl |
| $CF_3$ | $NO_2$ | $-CH_2-$ | 4-Cl |
| $CF_3$ | $NO_2$ | $-CH_2-$ | 2-F |
| $CF_3$ | $NO_2$ | $-CH_2-$ | 3-F |
| Cl | $NO_2$ | $-CH_2-$ | H |
| Cl | CN | $-CH_2-$ | 2-Cl |
| Cl | CN | $-CH_2-$ | 3-Cl |
| Cl | CN | $-CH_2-$ | 4-Cl |
| Cl | CN | $-CH_2-$ | 3-F |
| $C_2H_5O$ | $NO_2$ | $-CH_2-$ | H |
| $CH_3SO_2$ | $NO_2$ | $-CH_2-$ | H |
| $CF_3$ | $NO_2$ | $-CH_2CH_2-$ | H |
| $CF_3$ | $NO_2$ | $-(CH_2)_4-$ | H |
| $CF_3$ | $NO_2$ | $-CH_2-$ | 4-F |

**5.** Acylanilid, welches ausgewählt ist aus Verbindungen der Formel

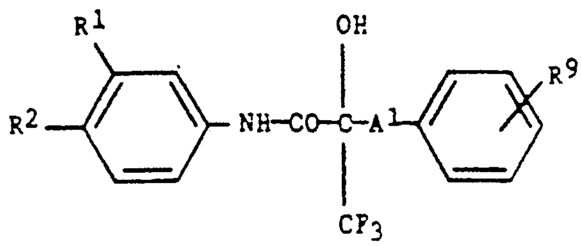

wobei die einzelnen Substituenten $R^1$, $R^2$, $A^1$ und $R^9$ in der folgenden Tabelle angegeben sind:

| $R^1$ | $R^2$ | $A^1$ | $R^9$ |
|---|---|---|---|
| Cl | CN | $-CH_2-$ | H |
| $CF_3$ | CN | $-CH_2-$ | H |
| $CF_3$ | CN | $-CH_2-$ | 2-Cl |
| $CF_3$ | CN | $-CH_2-$ | 2-F |
| $CF_3$ | CN | $-CH_2-$ | 3-F |
| $CF_3$ | CN | $-CH_2-$ | 4-F |
| $CF_3$ | $NO_2$ | $-CH_2-$ | H |
| $CF_3$ | $NO_2$ | $-CH_2-$ | 4-Cl |
| $CF_3$ | $NO_2$ | $-CH_2-$ | 2-F |
| $CF_3$ | $NO_2$ | $-CH_2-$ | 3-F |
| Cl | $NO_2$ | $-CH_2-$ | H |
| Cl | CN | $-CH_2-$ | 2-Cl |
| Cl | CN | $-CH_2-$ | 4-Cl |
| Cl | CN | $-CH_2-$ | 2-F |
| Cl | CN | $-CH_2-$ | 3-F |
| Cl | CN | $-CH_2-$ | 4-F |
| F | CN | $-CH_2-$ | H |
| F | CN | $-CH_2-$ | 4-F |
| CN | CN | $-CH_2-$ | H |
| H | CN | $-CH_2-$ | H |
| H | $NO_2$ | $-CH_2-$ | H |
| H | $NO_2$ | $-CH_2-$ | 2-Cl |
| $CF_3$ | $NO_2$ | $-(CH_2)_3$ | H |
| $CF_3$ | $NO_2$ | $-CH_2-$ | 4-F |

**6.** Die Verbindungen
3-Chloro-4-cyano-N-(2-hydroxy-3-p-methansulfonylphenyl-2-trifluoromethylpropionyl)anilin;
3-Chloro-4-cyano-N-(3-p-fluorophenyl-2-hydroxy-2-trifluoromethylpropionyl)anilin;
4-Cyano-3-trifluoromethyl-N-(3-p-chlorophenyl-2-hydroxy-2-trifluoromethylpropionyl)anilin;
4-Cyano-3-trifluoromethyl-N-(2-hydroxy-3-phenyl-2-trifluoromethylpropionyl)anilin;
4-Nitro-3-trifluoromethyl-N-(2-hydroxy-3-phenyl-2-trifluoromethylpropionyl)anilin;
4-Nitro-3-trifluoromethyl-N-(3-p-chlorophenyl-2-hydroxy-2-trifluoromethylpropionyl)anilin;
4-Nitro-3-trifluoromethyl-N-(3-p-fluorophenyl-2-hydroxy-2-trifluoromethylpropionyl)anilin;
4-Nitro-N-(3-p-fluorophenyl-2-hydroxy-2-trifluoromethylpropionyl)anilin;
4-Cyano-N-(2-hydroxy-2-trifluoromethyl-4-phenylbutyryl)anilin;
4-Nitro-3-trifluoromethyl-N-(3-o-chlorophenyl-2-hydroxy-2-trifluoromethylpropionyl)anilin;
4-Nitro-3-trifluoromethyl-N-(3-m-fluorophenyl-2-hydroxy-2-trifluoromethylpropionyl)anilin und
4-Cyano-3-trifluoromethyl-N-(3-p-fluorophenyl-2-hydroxy-2-trifluoromethylpropionyl)anilin.

**7.** Verfahren zur Herstellung eines Acylanilids nach Anspruch 1, bei welchem
(a) ein Amin der Formel

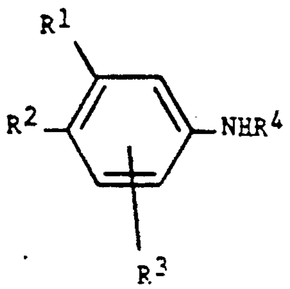

worin $R^1$, $R^2$, $R^3$ und $R^4$ die in Anspruch 1 angegebenen Bedeutungen besitzen, mit einer Säure der Formel

$HO_2C\text{-}CR^5R^6\text{-}A^1\text{-}R^7$

worin $R^5$, $R^6$, $R^7$ und $A^1$ die in Anspruch 1 angegebenen Bedeutungen besitzen, oder mit einem reaktiven Derivat dieser Säure umgesetzt wird;

(b) zur Herstellung eines erfindungsgemäßen Acylanilids, worin $R^4$ und $R^5$ unter Bildung einer Carbonyloxy-Gruppe miteinander verbunden, so daß ein Oxazolidindion gebildet wird, ein Isocyanat der Formel

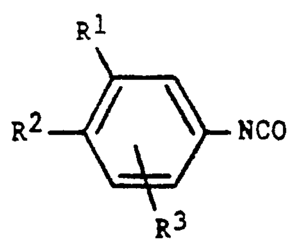

worin $R^1$, $R^2$ und $R^3$ die in Anspruch 1 angegebenen Bedeutungen besitzen, mit einem Ester der Formel

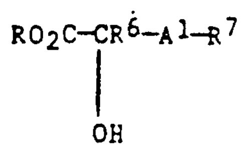

worin $R^6$, $R^7$ und $A^1$ die in Anspruch 1 angegebenen Bedeutungen besitzen und R für Alkyl mit bis zu 6 Kohlenstoffatomen steht, umgesetzt wird;

(c) zur Herstellung eines erfindungsgemäßen Acylanilids, worin $R^7$ für einen Heterocyclus und $A^1$ für Methylen steht, ein Epoxid der Formel

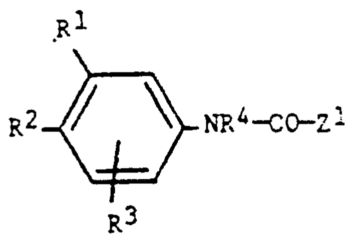

worin $R^1$, $R^2$, $R^3$ und $R^4$ die in Anspruch 1 angegebenen Bedeutungen besitzen und $Z^1$ die Formel

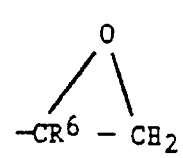

aufweist, worin $R^6$ die in Anspruch 1 angegebene Bedeutung besitzt, mit einem Heterocyclus oder einem reaktiven Derivat davon der Formel $R^7$-M, worin $R^7$ die unmittelbar vorstehend angegebene Bedeutung besitzt und M für ein Metallradikal steht, umgesetzt wird;

(d) zur Herstellung eines erfindungsgemäßen Acylanilids, worin $R^5$ für Hydroxy steht, das entsprechende Acylanilid, worin $R^5$ für Acyloxy steht, hydrolysiert wird;

(e) zur Herstellung eines erfindungsgemäßen Acylanilids, worin $R^5$ für Hydroxy und $R^4$ für Wasserstoff steht, das entsprechende Oxazolidindion hydrolysiert wird;

(f) zur Herstellung eines erfindungsgemäßen Acylanilids, worin $R^4$ für Alkyl steht, das entsprechende Acylanilid, worin $R^4$ für Wasserstoff steht, alkyliert wird;

(g) zur Herstellung eines erfindungsgemäßen Acylanilids, worin $R^5$ für Acyloxy steht, das entsprechende Acylanilid, worin $R^5$ für Hydroxy steht, acyliert wird;
(h) zur Herstellung eines erfindungsgemäßen Acylanilids, worin $R^4$ und $R^5$ unter Bildung einer Carbonyloxy-Gruppe miteinander verbunden sind, das entsprechende Acylanilid, worin $R^4$ für Wasserstoff und $R^5$ für Hydroxy steht, mit Phosgen ($COCl_2$) umgesetzt wird;
(i) zur Herstellung eines erfindungsgemäßen Acylanilids, worin eines oder mehrere der Symbole $R^1$ und $R^2$ oder ein Substituent in der Phenyl- oder heterocyclischen Gruppen $R^7$ aus Alkylsulfinyl, Perfluoroalkylsulfinyl oder Phenylsulfinyl oder aus Alkylsulfonyl, Perfluoroalkylsulfonyl oder Phenylsulfonyl besteht, das entsprechende Acylanilid, worin eines oder mehrere der Symbole $R^1$ und $R^2$ oder ein Substituent in der Phenyl- oder heterocyclischen Gruppe $R^7$ aus Alkylthio, Perfluoroalxylthio bzw. Phenylthio besteht, oxydiert wird; oder
(j) zur Trennung eines erfindungsgemäßen Acylanilids, worin $R^5$ für Hydroxy steht, in seine optischen Isomere ein Ester der Hydroxy-Gruppe $R^5$ mit einer optisch aktiven Säure hergestellt wird, die erhaltenen diastereoisomeren Ester durch fraktionierte Kristallisation oder Entspannungschromatographie getrennt werden und hierauf jeder gesonderte Ester in den Alkohol hydrolysiert wird.

**8.** Pharmazeutische Zusammensetzung, welche ein Acylanilid nach Anspruch 1 zusammen mit einem pharmazeutisch zulässigen Verdünnungs- oder Trägermittel enthält, wobei die Zusammensetzung gegebenenfalls ein oder mehrere Wirkstoffe enthält, die ausgewählt sind aus Antiöstrogenen, Progestinen, Inhibitoren der Gonodotrophinsekretion, cytotoxischen Mitteln, Antibiotika und antiinflammatorischen Mitteln.

**9.** Die Verwendung eines Acylanilids nach Anspruch 1 zur Herstellung eines Medikaments für die Erzielung eines antiandrogenen Effekts bei Warmblütern.

**10.** Die Verwendung eines Acylanilids nach Anspruch 4 zur Herstellung eines Medikaments für die Erzielung eines progestationalen Effekts bei Warmblütern.

**11.** Die Verwendung eines Acylanilids nach Anspruch 5 zur Herstellung eines Medikaments für die Erzielung eines antiprogestationalen Effekts bei Warmblütern.